# EUROPÄISCHE PATENTSCHRIFT

(11) **EP 2 059 493 B1**
(45) Veröffentlichungstag und Bekanntmachung des Hinweises auf die Patenterteilung: **16.11.2016**
(21) Anmeldenummer: 07803135.8
(22) Anmeldetag: 31.08.2007
(51) Int. Cl.: C07C 29/17, C07C 29/56, C07C 29/80, C07C 35/17, C07C 35/12, B01J 31/02, B01J 31/14, B01J 31/40

(54) **RÜCKGEWINNUNG VON PHENOL-LIGANDEN BEI DER HERSTELLUNG VON ISOPULEGOL**
RECOVERY OF PHENOL LIGANDS DURING THE PRODUCTION OF ISOPULEGOL
RECUPERATION DE LIGANDS PHENOL LORS DE LA PRODUCTION D'ISOPULEGOL

(30) Priorität: 01.09.2006 EP 06120012
(43) Veröffentlichungstag der Anmeldung: 20.05.2009
(73) Patentinhaber: BASF SE, 67056 Ludwigshafen am Rhein (DE)
(72) Erfinder: HEYDRICH, Gunnar, 67117 Limburgerhof (DE); GRALLA, Gabriele, 68161 Mannheim (DE); EBEL, Klaus, 68623 Lampertheim (DE); FRIEDRICH, Marko, 64653 Lorsch (DE)
(74) Vertreter: BASF IP Association
(86) Internationale Anmeldenummer: PCT/EP2007/059148
(87) Internationale Veröffentlichungsnummer: WO 2008/025851

(56) Entgegenhaltungen:
- EP-A- 1 225 163
- WO-A-2006/056435
- WO-A-2006/069659
- WO-A1-2006/092433

## Beschreibung

Die vorliegende Erfindung betrifft ein Verfahren zur Aufarbeitung eines Aluminium-haltigen Reaktionsprodukts aus der Herstellung von Isopulegol durch Cyclisierung von Citronellal in Gegenwart von Komplexverbindungen, umfassend wenigstens einen Liganden der Formel (I).

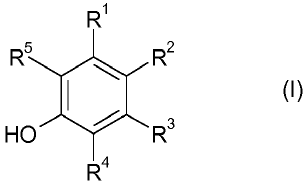

Darüber hinaus betrifft die Erfindung ein Verfahren zur Herstellung von Isopulegol sowie ein Verfahren zur Herstellung von Menthol.

Menthol stellt weltweit die mengenmäßig wichtigste Aromachemikalie dar. Der Bedarf an Menthol wird nach wie vor zu einem großen Teil durch Isolierung aus natürlichen Quellen gedeckt. Daneben existieren jedoch auch synthetische Zugänge zum Menthol, teils in racemischer Form, teils in Form des natürlichen Enantiomeren L-Menthol.

Ein wichtiges Zwischenprodukt zur Herstellung von racemischem wie optisch aktivem Menthol stellt Isopulegol dar, das üblicherweise durch cyclisierende Oxo-En-Reaktion von Citronellal in Gegenwart von Lewis-sauren Katalysatoren hergestellt wird und dabei in der Regel in Form von Gemischen der vier Diastereomere Isopulegol, iso-Isopulegol, neo-Isopulegol und neoiso-Isopulegol anfällt.

Als geeignete Katalysatoren zur Durchführung der vorstehend genannten Cyclisierung von Citronellal zu Isopulegol wurden sowohl heterogene Katalysatoren, wie SiO₂, Al₂O₃/SiO₂-, SiO₂/ZrO₂-, SiO₂/TiO₂-Mischkatalysatoren, Mordenite, Faujasite, Monmorillonite und Zeolithe - als auch homogene Katalysatoren, wie beispielsweise Sulfonsäuren oder Lewis-Säuren, wie beispielsweise SnCl₄, ZnCl₂ oder ZnBr₂ beschrieben.

Die EP-A 1 225 163 beschreibt die Cyclisierung von Citronellal zu Isopulegol in Gegenwart von Tris(2,6-diphenylphenol)-aluminium-Katalysatoren. Bei diesem Verfahren zur Cyclisierung von Citronellal zu Isopulegol werden teure und nur aufwändig herzustellende Katalysatorkomplexe eingesetzt. Nach dem beschriebenen, in homogener Phase durchzuführenden Verfahren wird der Katalysatorkomplex nach beendeter Reaktion hydrolysiert. Eine mögliche Wiedergewinnung und Wiedereinsetzbarkeit des dabei freigesetzten Liganden ist nicht beschrieben.

Aufgabe der vorliegenden Erfindung war es demnach, ein Verfahren bereitzustellen, das nach erfolgter Cyclisierung von Citronellal zu Isopulegol erlaubt, die eingesetzten Phenol-Liganden in hoher Reinheit und mit guter Ausbeute zurückzugewinnen. Darüber hinaus sollte dieses Verfahren insbesondere als kontinuierliches Verfahren geeignet sein.

Überraschenderweise wurde nun gefunden, dass sich die eingesetzten Phenol-Liganden nach erfolgter Cyclisierung von Citronellal durch destillative Auftrennung des Reaktionsprodukts unter Erhalt eines an Isopulegol angereicherten Kopfprodukts und eines an Isopulegol abgereicherten Sumpfprodukts und anschließendes Abtrennen des Liganden der Formel (I) aus dem Sumpfprodukt in hoher Reinheit und mit guter Ausbeute zurückgewinnen lassen. Darüber hinaus wurde gefunden, dass sich durch Inkontaktbringen des an Isopulegol abgereicherten Sumpfprodukts mit einer wässrigen Base unter Erhalt einer Aluminium-haltigen wässrigen Phase und einer die Hauptmenge der Liganden der Formel (I) enthaltenden organischen Phase die Reinheit des aus dem Abtrennen erhaltenen Liganden der Formel (I) steigern lässt. Insbesondere wurde gefunden, dass das Reaktionsprodukt aus der Cyclisierung von Citronellal ohne vorherige Hydrolyse der destillativen Auftrennung zugeführt werden kann.

Gegenstand der vorliegenden Erfindung ist somit ein Verfahren zur Aufarbeitung eines Aluminium-haltigen Reaktionsprodukts aus der Herstellung von Isopulegol durch Cyclisierung von Citronellal, enthaltend
i) Isopulegol,
ii) wenigstens einen Liganden der Formel (I), 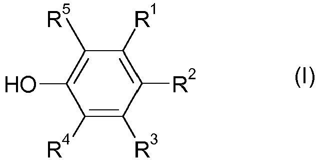 wobei
   R¹, R², R³ jeweils unabhängig voneinander ausgewählt sind unter Wasserstoff, Halogen, Nitro, C₁-C₈-Alkyl, C₁-C₈-Alkoxy, Di(C₁-C₄-alkyl)amino und gegebenenfalls substituiertem oder unsubstituiertem Aryl, und
   R⁴, R⁵ jeweils unabhängig voneinander ausgewählt sind unter Halogen, Nitro, C₁-C₈-Alkyl, C₁-C₈-Alkoxy, Di-(C₁-C₄-alkyl)amino und gegebenenfalls substituiertem oder unsubstituiertem Aryl oder Heteroaryl,
   in freier und/oder komplexgebundener Form,
   bei dem man
   a) das Reaktionsprodukt ohne vorherige Hydrolyse einer destillativen Auftrennung unter Erhalt eines an Isopulegol angereicherten Kopfprodukts und eines an Isopulegol abgereicherten Sumpfprodukts unterzieht,
   b) den Liganden der Formel (I) durch Kristallisation aus dem Sumpfprodukt abtrennt.

Eine spezielle Ausführungsform der vorliegenden Erfindung betrifft ein Verfahren wie zuvor beschrieben, bei dem man nach der destillativen Auftrennung in Schritt a) und vor der Abtrennung in Schritt b) das an Isopulegol abgereicherte Sumpfprodukt mit einer wässrigen Base unter Erhalt einer Aluminium-haltigen wässrigen Phase und einer die Hauptmenge der Liganden der Formel (I) enthaltenden organischen Phase in innigen Kontakt bringt (im Folgenden als Schritt a.2 bezeichnet).

Die nach dem erfindungsgemäßen Verfahren erhaltenen Phenol-Liganden der Formel (I) können, üblicherweise ohne weitere Aufreinigungsschritte, mit den entsprechenden Aluminiumverbindungen der Formeln (II) bzw. (III), wie im Folgenden definiert, wieder zu einem reaktiven Katalysatorkomplex umgesetzt werden. Bei den derartig wiederhergestellten Katalysatorkomplexen ist in der Regel keine bzw. keine nennenswerte Abschwächung der Reaktivität festzustellen.

Im Rahmen der vorliegenden Erfindung können den genannten Substituenten die nachfolgend beispielhaft genannten Bedeutungen zukommen:
C₁-C₈-Alkyl wie beispielsweise Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, Heptyl, Octyl, etc.;
C₁-C₈-Alkoxy wie beispielsweise Methoxy, Ethoxy, Propoxy, 1-Methylethoxy, Butoxy, 1-Methylpropoxy, 2-Methylpropoxy und 1,1-Dimethylethoxy, Pentoxy, 1-Methylbutoxy, 2-Methylbutoxy, 3-Methoxylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy, 1-Ethyl-2-methylpropoxy, Heptyloxy, Octyloxy, etc.;

Der Begriff "Halogen" steht im Rahmen der vorliegenden Erfindung für Fluor, Chlor, Brom und Jod, bevorzugt für Fluor und Chlor;

Die Reste R¹, R² und R³ haben z.B. unabhängig voneinander die folgenden Bedeutungen: Wasserstoff; Halogen wie beispielsweise Fluor, Chlor, Brom oder Iod; C₁-C₈-Alkyl wie Methyl, Ethyl, Propyl, Isopropyl, n-Butyl, Isobutyl, sec-Butyl, tert-Butyl, Pentyl, Hexyl, Heptyl, Octyl; C₁-C₈-Alkoxy wie Methoxy, Ethoxy, Propoxy, Isopropoxy, Butoxy, Isobutoxy, sec-Butoxy, tert-Butoxy, Pentoxy, Hexoxy, Heptoxy, Octoxy; beispielsweise durch C₁-C₄-Alkyl (z. B. Methyl, Ethyl, Propyl, Isopropyl, n-Butyl, iso-Butyl, sec-Butyl, tert-Butyl), C₁-C₄-Alkoxy (z. B. Methoxy, Ethoxy, Propoxy, Isopropoxy, Butoxy, Isobutoxy, sec-Butoxy, tert-Butoxy), Halogen (z. B. Fluor, Chlor, Brom oder Iod) und dergleichen mehr substituiertes Phenyl; Naphthyl substituiert mit 1 bis 7 Substituenten wie z. B. C₁-C₄-Alkyl (z.B. Methyl, Ethyl, Propyl, Isopropyl, n-Butyl, iso-Butyl, sec-Butyl, tert-Butyl), C₁-C₄-Alkoxy (z.B. Methoxy, Ethoxy, Propoxy, Isopropoxy, Butoxy, Isobutoxy, sec-Butoxy, tert-Butoxy), Halogen (z. B. Fluor, Chlor, Brom oder Iod) und dergleichen mehr; Di(C₁-C₄-alkyl)amino wie z. B. Dimethylamino, Diethylamino, Dipropylamino, Diisopropylamino, Dibutylamino; und Nitro.

Die Reste R⁴ und R⁵ können unabhängig voneinander die vorstehend für R¹, R² und R³ angegebenen Bedeutungen, ausgenommen Wasserstoff, besitzen. Des Weiteren können R⁴ und R⁵ unabhängig voneinander für Heteroaryl, insbesondere Furyl, Thienyl, Pyranyl, Benzofuryl, Isobenzofuryl, Benzothienyl, Indolyl, Isoindolyl, Carbazolyl, Pyridyl, Chinolyl, Isochinolyl oder Pyrazyl stehen. Die Heteroaryle können jeweils einen oder mehrere der vorstehend für Phenyl genannten Substituenten tragen.

Im Rahmen der vorliegenden Erfindung umfasst der Begriff "Ligand in freier Form" sowohl die freie Form des Liganden als auch sämtliche denkbaren Formen, die unter den Verfahrensbedingungen in die freie Form überführbar sind. Beispielhaft hierfür seien Alkoholate des Liganden genannt, die durch die basische Hydrolyse in die freie Form des Liganden überführt werden können.

Im Rahmen der vorliegenden Erfindung umfasst der Ausdruck "wässrige Base" allgemein wässrige Lösungen deren pH-Wert größer als 7 ist. Insbesondere handelt es sich um wässrige Lösungen von Alkali- und Erdalkalimetallhydroxiden, speziell wässrige Lösungen von KOH und NaOH.

Der Ausdruck "Aluminium-haltiges Reaktionsprodukt" beschreibt im Rahmen der vorliegenden Erfindung ein Reaktionsprodukt, das wenigstens eine Verbindung umfasst, die Aluminium ionisch, kovalent oder komplexgebunden enthält. Dabei handelt es sich um Verbindungen des Aluminiums, wie sie unter den Bedingungen des erfindungsgemäßen Verfahrens aus den bei der Cyclisierung von Citronellal eingesetzten Verbindungen der Formel (R¹⁴)₃₋ₚAlHₚ (II) oder MAlH₄ (III), wie hierunter definiert, resultieren.

Unter dem Ausdruck "Hauptmenge" soll im Rahmen der vorliegenden Erfindung ein prozentualer Mengenanteil an der vorhandenen Gesamtmenge einer Verbindung verstanden werden, der größer 50 %, bevorzugt größer 80 % und insbesondere bevorzugt größer 90 % ist.

Überraschenderweise wurde gefunden, dass Isopulegol aus den Aluminium-haltigen Reaktionsprodukten der Cyclisierung von Citronellal ohne vorherige Hydrolyse der jeweils als Katalysator eingesetzten Phenoxy-Aluminium-Verbindungen (gegebenenfalls nach der destillativen Entfernung eines eingesetzten Lösungsmittels und/oder zusätzlich eingesetzter Hilfsstoffe) in hohen Reinheiten abdestilliert werden kann. Dabei bilden sich im Destillationssumpf in der Regel keine erkennbaren unerwünschten oder störenden Nebenprodukte. In einer speziellen Ausführung erfolgt vor oder während der destillativen Auftrennung in Schritt a) die Zugabe eines geeigneten, inerten, höhersiedenden Lösungsmittels. Dann erhält man im Destillationssumpf eine Lösung des Liganden der Formel (I) in dem erwärmten, jeweils eingesetzten höhersiedenden Lösungsmittel.

Daher wird in dem erfindungsgemäßen Verfahren zur Aufarbeitung eines Aluminium-haltigen Reaktionsprodukts aus der Cyclisierung von Isopulegol das Reaktionsprodukt ohne vorherige Hydrolyse der destillativen Auftrennung in Schritt a) unterworfen.

### Schritt a):

In Schritt a) des erfindungsgemäßen Verfahrens wird das Reaktionsprodukt aus der Herstellung von Isopulegol durch Cyclisierung von Citronellal ohne vorherige Hydrolyse einer destillativen Auftrennung unter Erhalt eines an Isopulegol angereicherten Kopfprodukts und eines an Isopulegol abgereicherten Sumpfprodukts unterzogen.

In einer speziellen Ausführungsform wird in Schritt a) ein höher als das Isopulegol siedendes Lösungsmittel eingesetzt. Somit kann eine unerwünschte thermische Beanspruchung des Sumpfinhalts vermieden werden. Insbesondere liegen die darin enthaltenen Liganden der Formel (I) während der Abtrennung des Isopulegols nicht frei von Lösungsmittel vor. Das höhersiedende Lösungsmittel kann dem Reaktionsprodukt vor und/oder während der destillativen Auftrennung hinzugefügt werden. Vorzugsweise wird ein höhersiedendes Lösungsmittel eingesetzt, dessen Siedepunkt unter den Bedingungen der Destillation über dem Siedepunkt des Isopulegols liegt. Bevorzugt liegt der Siedepunkt des zugeführten Lösungsmittels unter den Bedingungen der Destillation wenigstens 5 °C, bevorzugt wenigstens 10 °C und insbesondere wenigstens 20 °C über dem Siedepunkt des Isopulegols.

Bevorzugte höhersiedende Lösungsmittel, die einen solchen Siedepunkt aufweisen, sind beispielsweise Kohlenwasserstoffe, wie Phenylcyclohexan, Phenyltoluol, Dibenzyltoluol, 1-Methylnaphthalin und Tridecan, 1-Decanol, 1,2-Propylencarbonat, Ether, wie Diethylenglycoldibutylether, Tetraethylenglycoldimethylether und Dibenzylether, sowie technische Gemische dieser Lösungsmittel. Insbesondere bevorzugt sind Gemische, die als Hauptbestandteil Phenylcyclohexan enthalten.

Bei Einsatz wenigstens eines höhersiedenden Lösungsmittels wird als an Isopulegol abgereichertes Sumpfprodukt in Schritt a) eine organische Phase erhalten, umfassend das höhersiedende Lösungsmittel, die Hauptmenge der Liganden der Formel (I) sowie gegebenenfalls wenigstens eine Aluminium-haltige Verbindung.

Vorzugsweise erfolgt die destillative Abtrennung von Isopulegol in Schritt a) bei einer Sumpftemperatur von höchstens 250 °C, bevorzugt höchstens 150 °C und besonders bevorzugt höchstens 100 °C. Zur Einhaltung dieser Maximaltemperaturen kann die Destillation gewünschtenfalls unter einem geeigneten Vakuum durchgeführt werden. Die untere Sumpftemperatur ist in der Regel unkritisch und beträgt im Allgemeinen wenigstens 0 °C vorzugsweise wenigstens 20 °C.

Der Druck in Schritt a) der erfindungsgemäßen Verfahren liegt unabhängig von der speziellen Ausführungsform im Allgemeinen in einem Bereich von 0,1 bis 1500 mbar, bevorzugt in einem Bereich von 1 bis 500 mbar und besonderst bevorzugt in einem Bereich von 5 bis 100 mbar.

Unabhängig von der Zusammensetzung des Reaktionsprodukts aus der Cyclisierung von Citronellal und vom Einsatz eines höhersiedenden Lösungsmittels kann die destillative Abtrennung des Isopulegols kontinuierlich oder diskontinuierlich, vorzugsweise kontinuierlich erfolgen. In einer geeigneten Vorgehensweise wird dem Reaktionsprodukt aus Schritt a) das höhersiedende Lösungsmittel vor der destillativen Auftrennung zugegeben und im Verlauf der Destillation die im Sumpf vorhandene Menge an höhersiedendem Lösungsmittel im Weiteren konstant gehalten.

Zur destillativen Auftrennung in Schritt a) können die üblichen dem Fachmann bekannten Vorrichtungen eingesetzt werden (siehe z. B. Sattler, Thermische Trennverfahren, 2. Auflage 1995, Weinheim, S. 135ff; Perry's Chemical Engineers Handbook, 7. Auflage 1997, New York, Section 13). Dazu zählen Destillationssäulen und -kolonnen, die mit Packungen, Einbauten etc. versehen sein können. Die eingesetzten Destillationssäulen können trennwirksame Einbauten enthalten, wie Trennböden, z. B. Lochböden, Glockenböden oder Ventilböden, geordnete Packungen, z. B. Blech- oder Gewebepackungen, oder regellose Schüttungen von Füllkörpern. Die in der/den eingesetzten Kolonne(n) notwendige Stufenzahl und das Rücklaufverhältnis richten sich im Wesentlichen nach den Reinheitsanforderungen und der relativen Siedelage der Bestandteile des Reaktionsprodukts aus der Herstellung von Isopulegol durch Cyclisierung von Citronellal und des höhersiedenden Lösungsmittels, wobei der Fachmann die konkreten Auslegungs- und Betriebsdaten nach bekannten Methoden ermitteln kann. Die destillative Auftrennung kann z. B. in einer oder mehreren miteinander gekoppelten Destillationskolonnen erfolgen.

Zur destillativen Auftrennung in Schritt a) eignen sich ebenfalls übliche Verdampfer, vorzugsweise Verdampfer mit Zwangsumlauf, besonders bevorzugt Fallfilmverdampfer.

In Abhängigkeit gegebenenfalls enthaltener zusätzlicher Komponenten im Reaktionsprodukt aus der Cyclisierung von Citronellal kann die Zusammensetzung des bei der destillativen Auftrennung erhaltenen Kopfprodukts es erforderlich machen, dieses gegebenenfalls einem weiteren Aufarbeitungsschritt zu unterziehen.

In einer speziellen Ausführungsform des erfindungsgemäßen Verfahrens zur Aufbereitung eines Reaktionsprodukts aus der Herstellung von Isopulegol durch Cyclisierung von Citronellal, enthält das Reaktionsprodukt zusätzlich ein niedriger siedendes Lösungsmittel (iii).

Der Ausdruck "niedriger siedendes Lösungsmittel (iii)" bezieht sich im Rahmen der vorliegenden Erfindung auf den Siedepunkt des Isopulegols. Insbesondere eignen sich hierfür solche Lösungsmittel oder Lösungsmittelgemische, die unter den Bedingungen der destillativen Auftrennung einen Siedepunkt aufweisen, der wenigstens 5 °C, bevorzugt 10 °C und insbesondere 20 °C unter dem des Isopulegols bei den jeweiligen Bedingungen liegt.

Bevorzugte Lösemittel mit einem solchen Siedepunkt sind im Rahmen der vorliegenden Erfindung inerte organischen Lösungsmittel oder Mischungen davon, wie beispielsweise aromatische Lösungsmittel, z. B. Toluol, Ethylbenzol oder Xylol, halogenierte Lösungsmittel, z. B. Dichlormethan , Dichlorethan oder Chlorbenzol, aliphatische Lösungsmittel, z. B. Pentan, Hexan oder Cyclohexan, Ether, z. B. Tetrahydrofuran, Diethylether, Methyl-tert-Butylether, Ester, z. B. Essigsäureethylester, oder Dimethylformamid (DMF), Dimethylsulfoxid (DMSO) und dergleichen mehr. Besonderst bevorzugt handelt es sich um Toluol.

Enthält das aufzuarbeitende Reaktionsprodukt ein solches niedriger siedendes Lösungsmittel, so wird dieses in einer geeigneten Ausführungsform vor der destillativen Abtrennung des Isopelugols zumindest teilweise aus dem Reaktionsprodukt entfernt. Die Abtrennung des niedriger siedenden Lösungsmittels erfolgt vorzugsweise ebenfalls destillativ. In Abhängigkeit vom Siedepunkt des niedriger siedenden Lösungsmittels können die üblichen zuvor genannten Destillationsvorrichtungen eingesetzt werden.

In einer weiteren geeigneten Ausführungsform erfolgt die destillative Auftrennung des Reaktionsprodukts in Schritt a) unter Erhalt eines an Isopulegol angereicherten Kopfprodukts, das gleichzeitig zumindest einen Teil, vorzugsweise die Hauptmenge des niedriger siedenden Lösungsmittels, enthält. In diesem Fall kann das Kopfprodukt einer weiteren Auftrennung, bevorzugt ebenfalls destillativ, unterzogen werden.

Das abgetrennte niedriger siedende Lösungsmittel wird mit Vorteil in die Cyclisierung des Citronellals zurückgeführt, in dem es als Lösungsmittel eingesetzt wird. Auf diese Weise erfordert das erfindungsgemäße Verfahren - bis auf Ergänzungen, die durch unvermeidliche Verluste erforderlich werden - die lediglich einmalige Bereitstellung einer Menge des niedriger siedenden Lösungsmittels.

In einer speziellen Ausführungsform des erfindungsgemäßen Verfahrens zur Aufbereitung eines Reaktionsprodukts aus der Herstellung von Isopulegol durch Cyclisierung von Citronellal, enthält das Reaktionsprodukt zusätzlich einen Hilfsstoff (iv).

Der Begriff "Hilfsstoff (iv)" bezieht sich im Rahmen der vorliegenden Erfindung auf Verbindungen, die bei der Cyclisierung von Citronellal zugesetzt werden, um unerwünschte Nebenreaktionen zu unterdrücken. Bevorzugt sind die Hilfsstoffe (iv) ausgewählt unter organischen Säuren, Carbonsäureanhydriden, Aldehyden, Ketonen und Vinylethern.

Speziell sind die Hilfsstoffe (iv) ausgewählt unter Säuren, vorzugsweise organische Säuren. Beispielhaft seien als organische Säuren genannt: Essigsäure, Propionsäure, Benzoesäure, Toluolsulfonsäure, Methansulfonsäure bevorzugt Essigsäure.

In einer weiteren speziellen Ausführungsform der vorliegenden Erfindung sind die Hilfsstoffe (iv) ausgewählt unter Carbonsäureanhydriden, Aldehyden, Ketonen und Vinylethern.

Die Hilfsstoffe (iv) der genannten Substanzklassen können jeweils einzeln oder in Form von Gemischen in dem aufzuarbeitenden Reaktionsprodukt vorliegen. Bevorzugte Gemische sind solche, die aus Verbindungen einer Substanzklasse bestehen. Besonders bevorzugt enthält das Reaktionsprodukt einen einzelnen Hilfsstoff.

Vorzugsweise werden die im Reaktionsprodukt aus der Cyclisierung von Citronellal enthaltenen Hilfsstoffe (iv) ebenfalls zumindest teilweise entfernt und soweit wie möglich in die Cyclisierung von Citronellal zurückgeführt.

Weisen die Hilfsstoffe (iv) unter den Bedingungen der Destillation einen Siedepunkt auf, der unterhalb oder nur geringfügig, d. h. weniger als 30 °C, über dem Siedepunkt des Isopulegols liegt, können diese durch Destillation aus der ausreagierten Mischung weitgehend und in dem Maße, in dem sie nicht gegebenenfalls selbst umgesetzt wurden, zurück gewonnen werden. In Abhängigkeit vom Siedepunkt des Hilfsstoffs können die üblichen zuvor genannten Destillationsvorrichtungen eingesetzt werden.

Weisen die Hilfsstoffe (iv) unter den Bedingungen der Destillation einen Siedepunkt auf, der deutlich oberhalb, d. h. wenigstens 30 °C, über dem Siedepunkt des Isopulegols liegt, verbleiben diese im Sumpfprodukt und werden gegebenenfalls in Schritt b) des erfindungsgemäßen Verfahrens entfernt, wenn ihre physikalischen Eigenschaften dies erlauben.

In einer weiteren geeigneten Ausführungsform erfolgt die destillative Auftrennung des Reaktionsprodukts in Schritt a) unter Erhalt eines an Isopulegol angereicherten Kopfprodukts, das gleichzeitig zumindest einen Teil, vorzugsweise die Hauptmenge des Hilfsstoffs (iv) enthält. Gegebenenfalls kann dieses Hauptprodukt ein niedriger siedendes Lösungsmittel, wie zuvor ausgeführt, enthalten. In diesem Fall kann das Kopfprodukt einer weiteren Auftrennung, bevorzugt ebenfalls destillativ, unterzogen werden.

Der abgetrennte Hilfsstoff (iv) wird, gegebenenfalls gemeinsam mit dem niedriger siedenden Lösungsmittel, mit Vorteil in die Cyclisierung des Citronellals zurückgeführt, in der er z. B. zur Unterdrückung unerwünschter Nebenreaktionen eingesetzt wird. Auf diese Weise erfordert das erfindungsgemäße Verfahren - bis auf Ergänzungen, die durch unvermeidliche Verluste erforderlich werden - die lediglich einmalige Bereitstellung einer Menge des Hilfsstoffs (iv).

Die Isopulegolabtrennung, die Zufuhr des höhersiedenden Lösungsmittels und gegebenenfalls die Leichtsiederabtrennung, d. h. die Abtrennung von gegebenenfalls vorhandenen Lösungsmitteln und flüchtigen Hilfsstoffen aus der Cyclisierung von Citronellal, können auf unterschiedliche Weisen kombiniert werden:

In einer geeigneten Ausführungsform verwendet man zur Destillation eine so genannte Trennwandkolonne, d. h. Zulaufstelle und ein Seitenabzug befinden sich auf entgegen gesetzten Seiten einer Trennwand, die sich über einen Abschnitt der Längsausdehnung der Kolonne erstreckt. Derartige Destillationskolonnen, die eine Trennwand enthalten, sind dem Fachmann an sich bekannt. Sofern sich Seitenabzug und Zulauf im Bereich der Trennwand befinden, entsteht eine zu einer Brugma- oder Peltyuk-Schaltung analoge Schaltung. Derartige Destillationen unter Verwendung von Trennwandkolonnen sind in der DE-A-33 02 525 und EP-A-0 804 951 beschrieben.

In diesem Fall kann z. B. als Kopfprodukt eine an Leichtsiedern angereicherte Fraktion und als Seitenabzug einen den Hauptteil an Isopulegol enthaltenden Strom abgezogen werden. Das höhersiedende Lösungsmittel wird unterhalb der Zulaufstelle, bevorzugt in den Sumpf der Kolonne oder kurz oberhalb des Sumpfes, zugefahren. Eine Lösung der Hauptmenge des Liganden der Formel (I) in dem höhersiedenden Lösungsmittel fällt als Sumpfprodukt an.

In einer alternativen Ausführungsform verwendet man zur Destillation gekoppelte Kolonnen. Diese Ausführungsform kann von Vorteil sein, wenn das Reaktionsprodukt der Cyclisierung von Citronellal ein Lösungsmittel und/oder einen flüchtigen Hilfsstoff enthält, wie im Folgenden näher ausgeführt.

In diesem Fall können Gemische aus Isopulegol und niedriger oder geringfügig höher siedenden Lösungsmitteln und/oder Hilfsstoff (iv) das Kopfprodukt der ersten Kolonne bilden und in der zweiten Kolonne einer Auftrennung unter Erhalt eines zumindest die Hauptmenge des Isopulegols enthaltenden Stroms und eines an Isopulegol abgereicherten, die niedriger siedenden Lösungsmittel und/oder Hilfsstoffe der Cyclisierung enthaltenden Stroms, unterzogen werden.

Ströme, die niedriger siedende Lösungsmittel (iii) und Hilfsstoff (iv) der Cyclisierung enthalten, können in der Regel ohne weitere Auftrennung in die Cyclisierung zurückgeführt werden.

Die Liganden der Formel (I) fallen, gegebenenfalls in Form ihrer Komplexe oder anderer Derivate, als Sumpfprodukt der ersten Kolonne an.

In einer bevorzugten Ausführungsform der vorliegenden Erfindung wird das in Schritt a) erhaltene an Isopulegol abgereicherte Sumpfprodukt vor der Abtrennung des Liganden der Formel (I) in Schritt b) mit einer wässrigen Base in Kontakt gebracht.

### Schritt a.2):

In Schritt a.2) des erfindungsgemäßen Verfahrens wird das an Isopulegol abgereicherte Sumpfprodukt mit einer wässrigen Base unter Erhalt einer Aluminium-haltigen wässrigen Phase und einer die Hauptmenge der Liganden der Formel (I) enthaltenden organischen Phase in innigen Kontakt gebracht. Bevorzugte wässrige Basen sind die oben genannten.

Das in Schritt a) erhaltene, an Isopulegol abgereicherte Sumpfprodukt kann neben dem Liganden der Formel (I) in freier oder komplexgebundener Form wenigstens eine weitere schwerflüchtige Komponente enthalten. Dazu zählen z. B. in Schritt a) zugesetzte höhersiedende Lösungsmittel, die Umsetzungsprodukte der zur Cyclisierung von Citronellal zu Isopulegol eingesetzten Aluminium-haltigen Verbindungen sowie gegebenenfalls in Schritt a) nicht abgetrennte Hilfsstoffe (iv). Da sich Aluminium-haltige Komponenten und/oder die Hilfsstoffe (iv) insbesondere bei einem kontinuierlichen Verfahren anreichern und sich speziell auf die Ausbeute und Reinheit der Abtrennung in Schritt b) negativ auswirken, ist es vorteilhaft, diese Verbindungen möglichst vollständig zu entfernen. Dies gilt speziell für die Aluminium-haltigen Verbindungen.

Das Inkontaktbringen in Schritt a.2) erfolgt vorzugsweise durch Extraktion. Die Zahl der Extraktionsstufen liegt vorzugsweise in einem Bereich von 1 bis 20 Stufen.

Als Extraktionsmittel dienen die zuvor genannten wässrigen Basen. Daher werden diese Ausdrücke im Rahmen der vorliegenden Erfindung synonym verwendet.

Zur Extraktion bringt man das an Isopulegol abgereicherte Sumpfprodukt aus Schritt a) mit einer wässrigen Base innig in Kontakt. Nach Trennung der Phasen erhält man eine die Hauptmenge des Liganden der Formel (I) enthaltende Phase und eine an Aluminium-haltigen Verbindungen angereicherte wässrige Phase. Anschließend entfernt man die wässrige Phase. Das Inkontaktbringen kann kontinuierlich oder diskontinuierlich erfolgen.

Zur diskontinuierlichen Durchführung bringt man unter mechanischer Bewegung, z. B. durch Rühren, das an Isopulegol abgereicherte Sumpfprodukt aus Schritt a) und das wässrige Extraktionsmittel in einem geeigneten Gefäß in Kontakt, lässt das Gemisch zur Phasentrennung ruhen und entfernt eine der Phasen, indem man zweckmäßigerweise die Phase mit der höheren Dichte am Boden des Gefäßes abzieht.

Mehrere diskontinuierliche Trennoperationen können kaskadenartig hintereinander durchgeführt werden, wobei die von der wässrigen Phase abgetrennte, die Hauptmenge des Liganden der Formel (I) enthaltende Phase jeweils mit einer frischen Portion des wässrigen Extraktionsmittels in Kontakt gebracht wird und/oder das wässrige Extraktionsmittel im Gegenstrom geführt wird.

Zur kontinuierlichen Durchführung der Extraktion führt man das wässrige Extraktionsmittel und den Strom des an Isopulegol abgereicherten Sumpfprodukts aus Schritt a) geeigneten Apparaturen in analoger Weise zur diskontinuierlichen Variante kontinuierlich zu. Gleichzeitig wird der Apparatur, in der die Trennung der Phasen stattfindet, ein Austrag der die Hauptmenge des Liganden der Formel (I) enthaltenden Phase und ein Austrag der an Aluminium-haltigen Verbindungen angereicherten wässrigen Phase kontinuierlich entnommen.

Die Extraktion erfolgt mindestens einstufig, z. B. in einer Mischer-Abscheider-Kombination. Geeignete Mischer sind sowohl dynamische als auch statische Mischer. Eine Extraktion in mehreren Stufen erfolgt beispielsweise in mehreren Mischer-Abscheidern oder Extraktionskolonnen.

In einer geeigneten Ausführungsform wird zur Verbesserung der Phasentrennung wenigstens eine Koalesziervorrichtung eingesetzt. Diese ist vorzugsweise ausgewählt unter Koaleszierfiltern, Elektrokoaleszern und Kombinationen davon. Beim Einsatz von Mischer-Abscheider-Vorrichtungen zur Extraktion hat sich der Einsatz von Koaleszierfiltern, wie Kerzen- oder Sandfiltern, als vorteilhaft zur Verbesserung der Phasentrennung herausgestellt. Der Filter kann dabei direkt nach dem Mischer (Rührbehälter) und/oder im organischen Ablauf des Abscheiders installiert werden. Des Weiteren bevorzugt zur Verbesserung der Phasentrennung ist der Einsatz von Elektrokoaleszern. Diese haben sich zur Abtrennung wässriger Fremdphasen von bis zu 5 Massen-% bewährt. Der Einsatz von Koalesziervorrichtungen eignet sich in dem erfindungsgemäßen Verfahren auch vorteilhaft zur Abscheidung von feindispergierter wässriger Phase aus dem die Hauptmenge des Liganden der Formel (I) enthaltenden organischen Austrag einer Extraktionskolonne.

In einer geeigneten Ausgestaltung erfolgt die Extraktion Aluminium-haltiger Komponenten aus dem an Isopulegol abgereicherten Sumpfprodukt aus Schritt a) in wenigstens einer Mischer-Abscheider-Kombination. Die Verwendung einer weiteren Mischer-Abscheider-Kombination ist insbesondere vorteilhaft, um Anteile des Liganden der Formel (I) oder gegebenenfalls des höhersiedenden Lösungsmittels, die gegebenenfalls mit den abzutrennenden Aluminium-haltigen Verbindungen teilweise in das Extraktionsmittel übergehen, nachträglich zu re-extrahieren und somit in das Verfahren zurückzuführen.

Unter bestimmten Umständen kann es vorteilhaft sein, die die Hauptmenge an Liganden der Formel (I) enthaltende organischen Phase vor dem Abtrennen in Schritt b) oder im Anschluss an das Abtrennen einem Trocknungsschritt zu unterziehen. Geeignete Trocknungsverfahren sind die üblichen, dem Fachmann bekannten, insbesondere die Adsorption an wasserentziehenden Mitteln, z. B. unter Verwendung eines zeolithischen Molekularsiebs.

In einer alternativen Ausführungsform des erfindungsgemäßen Verfahrens wird, nach dem Inkontaktbringen des an Isopulegol abgereicherten Sumpfprodukts mit der wässrigen Base, das Wasser destillativ entfernt.

Um ein frühzeitiges Abtrennen des Liganden der Formel (I) durch Fällung oder Kristallisation zu verhindern, sollte zu keinem Zeitpunkt während Schritt a.2) das Löslichkeitsprodukt des Liganden in der organischen Phase überschritten werden. Dies kann durch geeignete Wahl der Temperatur und/oder der Menge und Art gegebenenfalls zugegebener Lösungsmittel erfolgen.

Demzufolge wird in einer bevorzugten Ausführungsform des erfindungsgemäßen Verfahrens ein Austrag des erwärmten Sumpfprodukts aus Schritt a) mit einer erwärmten wässrigen Base in innigen Kontakt gebracht.

Der Ausdruck "erwärmt" bezeichnet im Rahmen der vorliegenden Erfindung eine Temperatur oberhalb der Raumtemperatur und unterhalb der jeweiligen Siedepunktstemperaturen der wässrigen oder organischen Lösungen bei den jeweiligen Reaktionsbedingungen. Insbesondere bezeichnet erwärmt eine Temperatur im Bereich von 25 bis 150 °C, speziell im Bereich von 70 bis 100 °C.

In Abhängigkeit der gegebenenfalls bei der Cyclisierung von Citronellal verwendeten Hilfsstoffe kann das an Isopulegol abgereicherte Sumpfprodukt gegebenenfalls weitere in Schritt a) nicht abgetrennte Komponenten enthalten. Diese werden vorzugsweise in Schritt a.2) abgetrennt. In diesem Fall kann man die erhaltene wässrige Phase einem geeigneten Trennverfahren unterziehen, um diese Komponenten, z. B. einen Hilfsstoff (iv), zurückzugewinnen.

In einer weiteren bevorzugten Ausführungsform des erfindungsgemäßen Verfahrens erfolgt das Abtrennen des Liganden der Formel (I) in Schritt b) unmittelbar aus dem in Schritt a) erhaltenen an Isopulegol abgereicherten Sumpfprodukt, d. h. ohne vorherige Aufreinigung durch Inkontaktbringen mit einer wässrigen Base, wie unter Schritt a.2) beschrieben. Für das direkte Abtrennen des Liganden aus dem in Schritt a) erhaltenen Sumpfprodukt gelten dieselben geeigneten und bevorzugten Ausführungsformen, wie sie im Folgenden für das Abtrennen aus der in Schritt a.2) erhaltenen organischen Phase ausgeführt werden.

### Schritt b):

In Schritt b) des erfindungsgemäßen Verfahrens wird der Ligand der Formel (I) aus der in Schritt a.2) erhaltenen die Hauptmenge des Liganden enthaltenden organischen Phase abgetrennt, wobei Schritt b) kontinuierlich oder diskontinuierlich ausgeführt werden kann. Das Abtrennen des Liganden der Formel (I) erfolgt durch Kristallisation.

Zur Kristallisation des Liganden der Formel (I) muss zunächst das Löslichkeitsprodukt des Liganden der Formel (I) in der organischen Phase aus Schritt a.2) überschritten werden. Dies kann beispielsweise durch einen Abkühlungsprozess der organischen Phase oder durch (teilweises) destillatives Abtrennen des Lösungsmittels erfolgen. Verfahren hierzu sind dem Fachmann bekannt. Zur technischen Ausgestaltung des erfindungsgemäßen Verfahrens sind beispielsweise übliche Kühlungskristallisatoren, Verdampfungskristallisatoren, Vakuumkristallisatoren, Kristallisierrinnen oder Sprühkristallisatoren geeignet.

In einer bevorzugten Ausführungsform des erfindungsgemäßen Verfahrens erfolgt die Kristallisation durch Abkühlen der organischen Phase aus Schritt a.2) des Verfahrens. Im Allgemeinen erfolgt die Kristallisation bei einer Temperatur im Bereich von -50 °C bis 100 °C, bevorzugt im Bereich von -20 °C bis 50 °C und speziell in einem Bereich von 10 °C bis 40 °C.

Dieser Prozess kann durch Hinzufügen von Impfkristallen beschleunigt werden.

Der kristalline Ligand der Formel (I) kann beispielsweise durch Filtration, Flotation, Zentrifugation oder Siebung aus der Lösung isoliert werden.

Der so zurückerhaltene Ligand der Formel (I) kann gegebenenfalls durch geeignete Trocknungsverfahren getrocknet werden. Verfahren hierfür sind dem Fachmann bekannt. Beispielsweise sind zur technischen Ausgestaltung des Verfahrens übliche Walzentrockner, Tellertrockner, Kammertrockner, Fließbetttrockner oder Strahlungstrockner geeignet.

Die an Ligand der Formel (I) abgereicherte organische Phase kann dem Verfahren erneut vor oder während Schritt a) zugeführt werden.

In einer geeigneten Ausführungsform des erfindungsgemäßen Verfahrens erfolgt die Kristallisation bei Abkühlen auf Raumtemperatur aus einer erwärmten, gesättigten, in Schritt a.2) erhaltenen organischen Phase oder entsprechend aus einem in Schritt a) erhaltenen erwärmten Sumpfprodukt.

In einer bevorzugten Ausführungsform des Verfahrens zur Aufarbeitung eines Reaktionsprodukts aus der Herstellung von Isopulegol ist der Ligand der Formel (I) ausgewählt unter 2,6-Diarylphenol-Liganden der Formel (I.a) 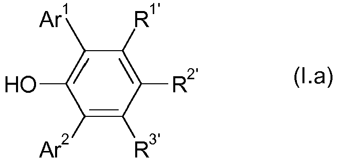 wobei
Ar¹ und Ar² jeweils unabhängig voneinander ausgewählt sind unter gegebenenfalls substituiertem oder unsubstituierten Aryl oder Heteroaryl und
R^{1'}, R^{2'}, R^{3'} jeweils unabhängig voneinander ausgewählt sind unter Wasserstoff, Halogen, Nitro, C₁-C₈-Alkyl, C₁-C₈-Alkoxy, gegebenenfalls substituiertem oder unsubstituiertem Aryl oder Di(C₁-C₄)-alkylamino.

In einer besonders bevorzugten Ausführungsform handelt es sich bei dem Liganden der Formel (I) um 2,6-Diphenylphenol.

Ein weiterer Gegenstand der vorliegenden Erfindung betrifft ein Verfahren zur Herstellung von Isopulegol der Formel (IV) 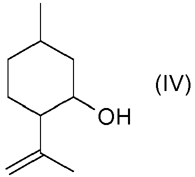 umfassend
α) die Cyclisierung von Citronellal der Formel (V) 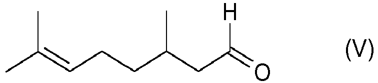 in Gegenwart eines tris(Phenoxy)-Aluminium-Katalysators, der erhältlich ist durch Umsetzung eines Liganden der Formel (I), bevorzugt eines tris(Biarylphenoxy)-Aluminium-Katalysators, der durch Umsetzung von Liganden der Formel (I.a) erhältlich ist, wie zuvor definiert,
   mit einer Aluminium-Verbindung der Formel (II),

   (R¹⁴)₃₋ₚAlHₚ (II)

   wobei
   - Al: Aluminium bedeutet,
   - R¹⁴: einen verzweigten oder unverzweigten Alkylrest mit 1 bis 5 Kohlenstoffatomen und
   - p: für 0 oder eine ganze Zahl von 1 bis 3 steht,
   und/oder
   mit einer Aluminium-Verbindung der Formel (III),

   MAlH₄ (III)

   wobei
   - Al: Aluminium bedeutet und
   - M: Lithium, Natrium oder Kalium bedeutet,
β) die Rückgewinnung des Liganden der Formel (I) nach erfolgter Umsetzung, indem man
   a) das in Schritt α) erhaltene Reaktionsprodukt ohne vorherige Hydrolyse einer destillativen Auftrennung unter Erhalt eines an Isopulegol angereicherten Kopfprodukts und eines an Isopulegol abgereicherten Sumpfprodukts unterzieht,
   b) den Liganden der Formel (I) durch Kristallisation aus dem Sumpfprodukt abtrennt.

Die in dem erfindungsgemäßen Verfahren verwendeten Katalysatoren und ihre Herstellung sind beispielsweise in der EP-A 1 225 163 beschrieben. Üblicherweise werden derartige Katalysatoren durch Umsetzung der entsprechenden 2,6-disubstituierten Phenolliganden mit geeigneten Aluminiumverbindungen, beispielsweise Trimethylaluminium, Triethylaluminium oder auch Diisobutylaluminiumhydrid gewonnen.

Eine bevorzugte Ausführungsform des zuvor beschriebenen erfindungsgemäßen Verfahrens zur Herstellung von Isopulegol der Formel (IV) umfasst die Rückgewinnung des Liganden der Formel (I) nach erfolgter Umsetzung,
indem man in Schritt β)
a) das in Schritt α) erhaltene Reaktionsprodukt ohne vorherige Hydrolyse einer destillativen Auftrennung unter Erhalt eines an Isopulegol angereicherten Kopfprodukts und eines an Isopulegol abgereicherten Sumpfprodukts unterzieht, zusätzlich
   a.2) das an Isopulegol abgereicherte Sumpfprodukts mit einer wässrigen Base unter Erhalt einer Aluminium-haltigen wässrigen Phase und einer die Hauptmenge der Liganden der Formel (I) enthaltenden organischen Phase in innigen Kontakt bringt und schließlich
b) den Liganden der Formel (I) durch Kristallisation aus der organischen Phase durch abtrennt.

Bezüglich der bevorzugten Ausführungsformen des erfindungsgemäßen Verfahrens zur Aufarbeitung eines Reaktionsprodukts aus der Herstellung von Isopulegol durch Cyclisierung von Citronellal sowie bezüglich der bevorzugten Liganden der Formel (I) wird im vollen Umfang auf die zuvor genannten bevorzugten Ausführungsformen verwiesen.

Die erfindungsgemäß als Katalysator verwendeten Phenoxy-Aluminium-Verbindungen erhält man beispielsweise, indem man die vorstehend beschriebenen Liganden der Formeln (I) bzw. (I.a) mit einer Aluminium-Verbindung der Formel (II)

(R¹⁴)₃₋ₚAlHₚ (II),

umsetzt. Dabei steht R¹⁴ für einen verzweigten oder unverzweigten Alkylrest mit 1 bis 5 Kohlenstoffatomen wie beispielsweise Methyl, Ethyl, Propyl, Isopropyl, Butyl, Isobutyl, Pentyl, Isopentyl oder Neopentyl. Der Index p steht für 0 oder eine ganze Zahl von 1 bis 3. Bevorzugt steht der Index p für 1 oder 0, besonders bevorzugt für 0. Bevorzugte Verbindungen der Formel (II) sind beispielsweise Trimethylaluminium, Triethylaluminium, Diisobutylaluminiumhydrid, besonders bevorzugt Trimethylaluminium und Triethylaluminium.

Alternativ dazu erhält man die erfindungsgemäß verwendeten Phenoxy-Aluminium-Verbindungen auch durch Umsetzung der Liganden der Formeln (I), bevorzugt der Formel (I.a) mit einer Aluminium-Verbindung der Formel (III)

MAlH₄ (III),

wobei M Lithium, Natrium oder Kalium bedeutet. Demzufolge eignen sich zur Herstellung der erfindungsgemäß verwendeten Phenoxy-Aluminium-Verbindungen durch Umsetzung der wie vorstehend beschriebenen Liganden der Formeln (I) bzw. (I.a) auch Lithiumaluminiumhydrid, Natriumaluminiumhydrid und Kaliumaluminiumhydrid sowie Gemische derselben. Darüber hinaus sind auch Gemische der genannten Verbindungen der Formeln (II) und (III) zur Herstellung erfindungsgemäß verwendeten Phenoxy-Aluminium-Verbindungen durch Umsetzung mit den vorstehend beschriebenen Liganden der Formeln (I) bzw. (I.a) geeignet.

Die Umsetzung wird vorteilhaft so durchgeführt, dass einer der vorstehend beschriebenen Liganden der Formeln (I) bzw. (I.a) mit einer Verbindung der Formel (II) oder (III) in Kontakt gebracht wird. Vorteilhaft führt man die Umsetzung in einem inerten organischen Lösungsmittel wie beispielsweise Toluol, Cyclohexan, Dichlormethan, Xylol, Ethylbenzol, Chlorbenzol, Tetrahydrofuran, Diethylether, Methyl-tert-Butylether, Essigsäureethylester, Pentan, Hexan, Dichlorethan, Dimethylformamid (DMF), Dimethylsulfoxid (DMSO) und dergleichen mehr durch, wobei der Einsatz vorgetrockneter bzw. wasserfreier Lösungsmittel als besonders vorteilhaft anzusehen ist. Üblicherweise erfolgt die Umsetzung bei Temperaturen im Bereich von etwa -100 °C bis etwa 100 °C, bevorzugt bei etwa -50 °C bis etwa 50 °C, besonders bevorzugt bei etwa -30 °C bis etwa 30 °C.

Bei der Herstellung der erfindungsgemäß verwendeten Phenoxy-Aluminium-Verbindungen reagieren die phenolischen Hydroxy-Gruppen der eingesetzten Liganden der Formeln (I) bzw. (I.a) mit der bzw. den Verbindungen der Formeln (II) und (III). Theoretisch kann jedes Aluminium-Atom mit 1 bis 3 phenolischen Hydroxy-Gruppen reagieren. Aufgrund der sterischen Eigenschaften bzw. Anforderungen der eingesetzten Liganden der Formeln (I) bzw. (I.a) kann es dabei zur Ausbildung höhermolekularer Strukturen wie linearen Strukturen oder Netzwerken kommen.

Vorteilhaft wählt man dabei das molare Verhältnis der eingesetzten Liganden der Formeln (I) bzw. (I.a) zu den eingesetzten Verbindungen der Formel (II) und/oder (III) so, dass die Menge an nicht abreagierten Verbindungen der Formeln (II) und/oder (III) möglichst gering ist. Vorzugsweise wählt man das genannte Verhältnis so, dass nach dem Inkontaktbringen der Liganden der Formeln (I) bzw. (I.a) mit der bzw. den Verbindungen der Formeln (II) und (III) keine nicht umgesetzte Verbindung der Formel (II) und/oder (III) mehr vorliegt. Unter Berücksichtigung des wirtschaftlichen Aspekts ist es empfehlenswert, den Überschuss der eingesetzten Liganden der Formeln (I) bzw. (I.a) gering zu halten. Besonders bevorzugt setzt man Liganden der Formeln (I) bzw. (I.a) und die Verbindungen der Formeln (II) und/oder (III) in einem molaren Verhältnis von etwa 4:1 bis etwa 1:1, ganz besonders bevorzugt von etwa 3:1 bis etwa 1,5:1 und am meisten bevorzugt im molaren Verhältnis von etwa 1,5:1 ein.

Zur Herstellung der erfindungsgemäß verwendeten Phenoxy-Aluminium-Verbindungen geht man im Rahmen einer bevorzugten Ausführungsform der vorliegenden Erfindung so vor, dass man, je nach Löslichkeit, eine etwa 0,001 bis etwa 1 molare Lösung des gewählten Liganden der Formel (I) bzw. (I.a) in einem geeigneten organischen Lösungsmittel, beispielsweise Toluol, bei einer Temperatur von etwa -10 bis etwa 30 °C vorlegt und eine Aluminiumverbindung der Formel (II) und/oder (III), vorzugsweise in Form einer Lösung, beispielsweise eine Lösung von Trimethyl- oder Triethylaluminium in Toluol, zugibt.

Die Reaktion zwischen den eingesetzten Liganden der Formel (I) bzw. (I.a) und den Aluminiumverbindungen der Formeln (II) und/oder (III) erfolgt in der Regel rasch und ist meist, in Abhängigkeit von den gewählten Reaktionsbedingungen, nach etwa 10 min bis etwa 2 h, oft nach etwa 1 h, abgeschlossen. Bei Einsatz reaktionsträgerer Reaktanden kann es vorteilhaft sein, die Temperatur des Reaktionsgemisches kurzzeitig zu erhöhen.

In Abhängigkeit von den gewählten Reaktionsbedingungen, insbesondere im Hinblick auf die Löslichkeit der umzusetzenden Liganden der Formel (I) bzw. (I.a) und der Aluminiumverbindung der Formel (II) und/oder (III) in den gewählten Lösungsmitteln, den Konzentrationen sowie den Reaktionstemperaturen erhält man die erfindungsgemäß verwendeten Phenoxy-Aluminium-Verbindungen in Form eines Feststoffes, einer Suspension oder einer Lösung im eingesetzten Lösungsmittel bzw. Lösungsmittelgemisch. Die so erhaltenen erfindungsgemäß verwendeten Phenoxy-Aluminium-Verbindungen können in der jeweils erhaltenen Form weiterverwendet werden oder abgetrennt und von den eingesetzten Lösungsmitteln befreit werden.

Die Isolierung kann dabei nach dem Fachmann bekannten und vorteilhaft erscheinenden Methoden erfolgen. Vorzugsweise führt man die Isolierung, Lagerung bzw. Weiterbehandlung der erfindungsgemäß verwendeten Phenoxy-Aluminium-Verbindungen unter weitgehendem Ausschluss von Sauerstoff und Feuchtigkeit durch.

Zur Durchführung des erfindungsgemäßen Verfahrens zur Herstellung von Isopulegol geht man vorteilhaft so vor, dass man zunächst eine Lösung der erfindungsgemäß verwendeten Phenoxy-Aluminium-Verbindungen in einem geeigneten Lösungsmittel, wie vorstehend beschrieben, bereitstellt. Dieser Lösung setzt man dann erfindungsgemäß das zu cyclisierende racemische oder nicht-racemische Citronellal zu. Das Citronellal kann dabei als solches oder in Form einer Lösung, vorteilhaft in einem der vorstehend genannten geeigneten Lösungsmittel zugegeben werden. Im Rahmen einer bevorzugten Ausführungsform des erfindungsgemäßen Verfahrens bereitet man zunächst eine Lösung des gewählten Liganden der Formeln (I) bzw. (I.a) in Toluol und setzt dann, vorteilhaft unter Rühren, die gewählte Aluminium-Verbindung der Formel (II) und/oder (III), bevorzugt Trimethyl- oder Triethylaluminium in toluolischer Lösung, zu.

Als Ausgangsstoff zur Durchführung des erfindungsgemäßen Cyclisierungsverfahrens eignet sich Citronellal, das nach jedwedem Verfahren hergestellt sein kann. Bevorzugt setzt man Citronellal ein, das eine Reinheit von etwa 90 bis etwa 99,9 Gew.-%, besonders bevorzugt von etwa 95 bis etwa 99,9 Gew.-% aufweist.

Die Zugabe des zu cyclisierenden Citronellals erfolgt vorteilhaft bei Temperaturen im Bereich von etwa -40 °C bis etwa 40 °C, bevorzugt im Bereich von etwa -20 °C bis etwa 20 °C. Dazu wird vorteilhaft die bereitete Lösung der erfindungsgemäß verwendeten Phenoxy-Aluminium-Verbindung auf eine Temperatur in diesem Bereich, z. B. auf eine Temperatur im Bereich von -10 °C bis 10 °C, abgekühlt und vorgekühltes Citronellal bzw. eine vorgekühlte Lösung von Citronellal zugegeben.

Die Zugabe des Citronellals bzw. der Lösung davon kann so vorgenommen werden, dass man entweder die gesamte Menge auf einmal zusetzt oder sie portionsweise oder auch kontinuierlich zur bereiteten Katalysatorlösung gibt. Als Lösungsmittel eignen sich wiederum die vorstehend genannten Lösungsmittel, insbesondere Toluol. Bevorzugt setzt man das zu cyclisierende Citronellal als solches, d. h. ohne weiteren Zusatz von Lösungsmitteln, ein. Bei Einsatz eines Lösungsmittels wird die gesamte Lösungsmittelmenge (für Katalysatorherstellung und zur Durchführung der Cyclisierungsreaktion) vorteilhaft so gewählt, dass das volumenbezogene Verhältnis von umzusetzendem Citronellal zum Lösungsmittel etwa 2:1 bis etwa 1:20, bevorzugt von etwa 1,5:1 bis etwa 1:10 beträgt.

Das Mengenverhältnis zwischen dem umzusetzenden Citronellal und der eingesetzten Menge der erfindungsgemäß verwendeten Phenoxy-Aluminium-Verbindung wird durch die Menge der zur Herstellung derselben eingesetzten Verbindungen der Formel (I) bzw. (I.a) und der Formel (II) und/oder (III), also durch das Mengenverhältnis von eingesetztem Ligand zu eingesetzter Aluminium-Verbindung der Formel (II) und/oder (III), bestimmt.

Erfindungsgemäß wählt man die Menge von umzusetzendem Citronellal zur eingesetzten Menge an Aluminium-Verbindung der Formel (II) und/oder (III) so, dass das molare Verhältnis etwa 5:1 bis etwa 1000:1, bevorzugt etwa 10:1 bis etwa 500:1, besonders bevorzugt etwa 50:1 bis etwa 200:1 beträgt.

Unabhängig davon kann das Verhältnis zwischen eingesetztem Ligand der Formel (I) bzw. (I.a) und der eingesetzten Aluminium-Verbindung der Formel (II) und/oder (III) in den vorstehend zur Herstellung der erfindungsgemäßen Phenoxy-Aluminium-Verbindung genannten Grenzen variiert werden.

Die Cyclisierung von Citronellal zu Isopulegol erfolgt je nach Wahl der Reaktionspartner und Reaktionsbedingungen in der Regel rasch und ist üblicherweise nach etwa 0,5 bis etwa 10 h, oft nach etwa 5 h, weitgehend abgeschlossen. Der Reaktionsfortschritt kann durch dem Fachmann an sich bekannte Methoden, beispielsweise durch chromatographische, speziell gaschromatographische Methoden oder auch HPLC-Methoden leicht verfolgt werden.

Im Rahmen einer bevorzugten Ausführungsform des erfindungsgemäßen Verfahrens führt man die Cyclisierung von Citronellal zu Isopulegol in Gegenwart eines Hilfsstoffs (iv), beispielsweise einer Säure, vorzugsweise einer organischen Säure, durch. Beispielhaft seien als vorteilhaft einsetzbare organische Säuren genannt: Essigsäure, Propionsäure, Benzoesäure, Toluolsulfonsäure, Methansulfonsäure, bevorzugt Essigsäure. Die genannten Säuren werden vorteilhaft in einer Menge von etwa 0,5 bis etwa 10 Gew.-%, bezogen auf die Menge an umzusetzendem Citronellal, eingesetzt. Vorteilhaft werden sie zusammen mit dem Citronellal, z. B. in Form eines Gemisches, dem Reaktionsgemisch zugesetzt.

In einer besonders bevorzugten Ausführungsform führt man das erfindungsgemäße Verfahren zur Herstellung von Isopulegol durch Cyclisierung von Citronellal in Gegenwart mindestens eines Hilfsstoffs (iv) durch, der ausgewählt ist unter Carbonsäureanhydriden, Aldehyden, Ketonen und Vinylethern.

Die Hilfsstoffe (iv) der genannten Substanzklassen können jeweils einzeln oder in Form von Gemischen untereinander eingesetzt werden. Bevorzugt setzt man im Fall von Gemischen solche ein, die aus Verbindungen einer Substanzklasse bestehen. Besonders bevorzugt setzt man einzelne Verbindungen ein. Unter wie nachfolgend beschriebenem Einsatz der genannten Verbindungen gelingt es in der Regel, die Bildung unerwünschter Nebenprodukte weitgehend zu unterdrücken.

Im Rahmen einer bevorzugten Ausführungsform führt man die Cyclisierung von Citronellal in Gegenwart eines Carbonsäureanhydrids der Formel (VI) 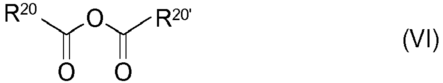 durch, wobei die Reste R²⁰ und R^{20'} gleich oder verschieden, bevorzugt gleich, sein können und ausgewählt sind unter verzweigtem oder unverzweigtem C₁-C₁₂-Alkyl oder C₇-C₁₂-Aralkyl oder C₆-C₁₀-Aryl, wobei die genannten Reste jeweils einen oder mehrere, in der Regel 1 bis etwa 3, gleiche oder verschiedene Substituenten, ausgewählt unter OR¹⁰, SR¹⁰, NR⁸R⁹ und Halogen, aufweisen können und wobei R²⁰ und R^{20'} gemeinsam auch einen 5- bis 8-gliedrigen Ring bilden können, der eine oder mehrere ethylenische Doppelbindungen und ein oder mehrere gleiche oder verschiedene Heteroatome, ausgewählt aus der Gruppe O, S und NR^{11b} aufweisen kann und wobei R⁸, R⁹, R¹⁰ und R¹¹ jeweils unabhängig voneinander ausgewählt sind unter C₁-C₆-Alkyl, C₇-C₁₂-Aralkyl oder gegebenenfalls substituiertem oder unsubstituiertem C₆-C₁₀-Aryl.

Im Rahmen einer weiteren bevorzugten Ausführungsform führt man die Cyclisierung von Citronellal in Gegenwart eines Aldehyds der Formel (VII) 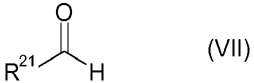 durch, wobei der Rest R²¹ ausgewählt ist unter verzweigtem oder unverzweigtem C₁-C₁₂-Alkyl, C₇-C₁₂-Aralkyl oder C₆-C₁₀-Aryl, wobei die genannten Reste jeweils einen oder mehrere, bevorzugt 1 bis 3, gleiche oder verschiedene Substituenten, ausgewählt unter OR¹⁰, SR¹⁰ NR⁸R⁹ und Halogen, aufweisen können, wobei R⁸, R⁹ und R¹⁰ jeweils unabhängig voneinander ausgewählt sind unter C₁-C₆-Alkyl, C₇-C₁₂-Aralkyl oder gegebenenfalls substituiertem oder unsubstituiertem C₆-C₁₀-Aryl.

Im Rahmen einer weiteren bevorzugten Ausführungsform führt man Cyclisierung von Citronellal in Gegenwart eines Ketons der Formel (VIII) 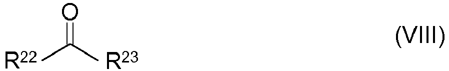 durch, wobei die Reste R²² und R²³ jeweils gleich oder verschieden sein können und ausgewählt sind unter verzweigtem oder unverzweigtem C₁-C₁₂-Alkyl, C₇-C₁₂-Aralkyl, C₆-C₁₀-Aryl oder C₁-C₆-Alkoxycarbonyl, wobei die genannten Reste jeweils einen oder mehrere, bevorzugt 1 bis 3, gleiche oder verschiedene Substituenten, ausgewählt unter OR¹⁰, SR¹⁰ NR⁸R⁹ und Halogen aufweisen können, und wobei R²² und R²³ gemeinsam auch einen 5- bis 8-gliedrigen Ring bilden können, der eine oder mehrere ethylenische Doppelbindungen und ein oder mehrere gleiche oder verschiedene Heteroatome, ausgewählt unter O, S, NR¹¹ aufweisen kann und wobei R⁸, R⁹, R¹⁰ und R¹¹ jeweils unabhängig voneinander ausgewählt sind unter C₁-C₆-Alkyl, C₇-C₁₂-Aralkyl oder gegebenenfalls substituiertem oder unsubstituiertem C₆-C₁₀-Aryl.

Alternativ zu den zuvor genannten Carbonylverbindungen lassen sich auch Vinylether der allgemeinen Formel (IX) 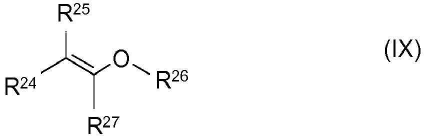 im Rahmen des erfindungsgemäßen Verfahrens einsetzen, wobei die Reste R²⁴, R²⁵, R²⁶ und R²⁷ unabhängig voneinander jeweils gleich oder verschieden sein können und ausgewählt sind unter verzweigtem oder unverzweigtem C₁-C₁₂-Alkyl, C₇-C₁₂-Aralkyl oder C₆-C₁₀-Aryl, wobei die genannten Reste jeweils einen oder mehrere, bevorzugt 1 bis 3, gleiche oder verschiedene Substituenten, ausgewählt unter Oxo, OR¹⁰, SR¹⁰ NR⁸R⁹ und Halogen aufweisen können und wobei R²⁵ und R²⁶ gemeinsam auch einen 5- bis 8-gliedrigen Ring bilden können, der eine oder mehrere ethylenische Doppelbindungen und ein oder mehrere, üblicherweise 1 oder 2, gleiche oder verschiedene Heteroatome, ausgewählt unter O, S, NR¹¹ aufweisen kann und wobei R⁸, R⁹, R¹⁰ und R¹¹ jeweils unabhängig voneinander ausgewählt sind unter C₁-C₆-Alkyl, C₇-C₁₂-Aralkyl oder gegebenenfalls substituiertem oder unsubstituiertem C₆-C₁₀-Aryl.

Dabei seien für die genannten Reste R⁷ bis R¹¹ beispielhaft die folgenden Bedeutungen angegeben: C₁-C₆-Alkyl wie z. B. Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1 ,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl; C₇-C₁₂-Aralkyl wie z. B. Benzyl, 1-Phenylethyl, 2-Phenylethyl; C₆-C₁₀-Aryl wie Phenyl oder Naphthyl.

Im Rahmen einer bevorzugten Ausführungsform des erfindungsgemäßen Verfahrens führt man die Cyclisierung von Citronellal in Gegenwart eines Carbonsäureanhydrids der Formel (VI) durch, wobei die Reste R²⁰ und R^{20'} gleich sind und ausgewählt sind unter verzweigtem oder unverzweigtem C₁-C₁₂-Alkyl, C₇-C₁₂-Aralkyl oder C₆-C₁₀-Aryl, und wobei R²⁰ und R²⁰ gemeinsam auch einen 5- bis 8-gliedrigen Ring bilden können, der eine oder mehrere ethylenische Doppelbindungen und ein oder mehrere gleiche oder verschiedene Heteroatome ausgewählt unter O, S, NR¹¹ aufweisen kann, worin R¹¹ eine der zuvor gegebenen Bedeutungen haben kann.

Insbesondere bevorzugt setzt man solche Carbonsäureanhydride ein, bei denen die Reste R²⁰ und R^{20'} gleich sind und ausgewählt sind unter verzweigtem oder unverzweigtem C₁-C₁₂-Alkyl oder C₆-C₁₀-Aryl. Beispielhaft seien als erfindungsgemäß besonders bevorzugt einzusetzende Carbonsäureanhydride genannt: Essigsäureanhydrid, Propionsäureanhydrid, Pivalinsäureanhydrid und Benzoesäureanhydrid.

Als erfindungsgemäß ebenfalls bevorzugt einsetzbare Aldehyde der Formel (VII) seien beispielhaft Acetaldeyhd, Propionaldehyd und Chloral (Trichloracetaldehyd) genannt.

Führt man die Cyclisierung von Citronellal im Rahmen einer weiteren bevorzugten Ausführungsform in Gegenwart eines Ketons der Formel (VIII) durch, setzt man mit Vorteil solche mit einer aktivierten, d. h. elektronenarmen Carbonylfunktion, ein. Beispielhaft seien die folgenden Ketone genannten, die sich in besonderem Maße zum Einsatz im Rahmen des erfindungsgemäßen Verfahren eigenen: 1,1,1-Trifluoraceton, 1,1,1-Trifluoracetophenon, Hexafluoraceton, Brenztraubensäuremethylester und Brenztraubensäureethylester.

Als erfindungsgemäß ebenfalls bevorzugt einsetzbare Vinylether der Formel (IX) seien beispielhaft genannt: Methylvinylether, Ethylvinylether, Isobutylvinylether und 3,4-Dihydro-2H-pyran.

Die genannten Verbindungsklassen können gleichermaßen mit gutem Erfolg im Rahmen dieser bevorzugten Ausführungsform des erfindungsgemäßen Verfahrens eingesetzt werden. Im Hinblick auf praktische Gesichtspunkte wie beispielsweise eine höhere Reaktionsgeschwindigkeit hat sich der Einsatz von Aldehyden und/oder elektronenarmen Ketonen als vorteilhaft erweisen.

Die Menge an erfindungsgemäß zu verwendendem Carbonsäureanhydrid, Aldehyd, Keton und/oder Vinylether kann in breiten Grenzen variiert werden und richtet sich nach Art des eingesetzten Stoffes und dem Reinheitsgrad bzw. dem Vorhandensein noch nicht näher identifizierter Verunreinigungen. Üblicherweise setzt man die genannten Verbindungen bzw. deren Gemische in einer Menge von etwa 0,01 mol-% bis etwa 5 mol-%, bevorzugt von etwa 0,1 mol-% bis etwa 2 mol-%, bezogen auf die eingesetzte Menge an Citronellal, ein.

An die Art und Weise der Reaktionsführung, beispielsweise die Ausgestaltung von Reaktoren oder die Reihenfolge der Zugabe einzelner Reaktionspartner, sind keine besonderen Anforderungen zu stellen, solange eine Reaktionsführung unter weitgehendem Ausschluss von Sauerstoff und Wasser gewährleistet ist.

Zur Durchführung des erfindungsgemäßen Verfahrens im Rahmen dieser bevorzugten Ausführungsform geht man vorteilhaft so vor, dass man zunächst eine Lösung der erfindungsgemäß einzusetzenden Phenoxy-Aluminium-Verbindung in einem geeigneten Lösungsmittel wie vorstehend beschrieben bereitstellt. Dieser Lösung setzt man dann erfindungsgemäß bevorzugt ein Gemisch des zu cyclisierenden racemischen oder nicht-racemischen Citronellals mit dem gewählten Carbonsäureanhydrid, dem Aldehyd, dem aktivierten Keton und/oder dem Vinylether zu. Alternativ dazu kann man beispielsweise auch die Lösung der erfindungsgemäß einzusetzenden Phenoxy-Aluminium-Verbindung zunächst mit dem gegebenenfalls jeweils gewählten Carbonsäureanhydrid, dem Aldehyd, dem Keton und/oder dem Vinylether versetzen und im Anschluss daran das zu cyclisierende Citronellal zugeben.

Es hat sich als vorteilhaft erwiesen, das Citronellal bzw. das Gemisch von Citronellal mit der gewählten Verbindung innerhalb eines Zeitraums von etwa 30 min bis etwa 6 h, bevorzugt innerhalb von etwa 2 h bis etwa 4 h, zur Katalysatorlösung bzw. dem Reaktionsgemisch zuzudosieren. Das Citronellal kann dabei als solches oder in Form einer Lösung, vorteilhaft in einem der vorstehend genannten geeigneten Lösungsmittel zugegeben werden. Im Rahmen einer wiederum bevorzugten Ausführungsform des erfindungsgemäßen Verfahrens bereitet man zunächst eine Lösung des gewählten Liganden der Formeln (I) bzw. (I.a) in Toluol und setzt dann, zweckmäßigerweise unter Rühren, die gewählte Aluminium-Verbindung der Formel (II) und/oder (III), bevorzugt Trimethyl- oder Triethylaluminium in toluolischer Lösung, zu.

Die Zugabe des zu cyclisierenden Citronellals bzw. des Gemischs von Citronellal mit dem gewählten Carbonsäureanhydrid, Aldehyd, aktivierten Keton und/oder dem Vinylether erfolgt im Rahmen dieser Ausführungsform vorteilhaft bei Temperaturen im Bereich von etwa -40 °C bis etwa 40 °C, bevorzugt im Bereich von etwa -20 °C bis etwa 20 °C. Dazu wird vorteilhaft die bereitete Lösung oder Suspension der erfindungsgemäßen Phenoxy-Aluminium-Verbindung auf eine Temperatur in diesem Bereich, z. B. auf eine Temperatur im Bereich von -10 °C bis 10 °C, abgekühlt und die weiteren Reaktanden in vorgekühlter Form zugegeben.

Die Zugabe des Gemischs von Citronellals und der gewählten weiteren Verbindung kann so vorgenommen werden, dass man entweder die gesamte Menge Citronellal auf einmal zusetzt oder sie portionsweise oder auch kontinuierlich zur bereiteten Katalysatorlösung gibt. Als Lösungsmittel eignen sich wiederum bevorzugt die vorstehend genannten Lösungsmittel, insbesondere Toluol. Bevorzugt setzt man das zu cyclisierende Citronellal in Form eines Gemisches mit dem gewählten Carbonsäureanhydrid, Aldehyd, aktivierten Keton und/oder Vinylether ohne weiteren Zusatz von Lösungsmitteln ein. Bei Einsatz eines Lösungsmittels wählt man die gesamte Lösungsmittelmenge vorteilhaft so, dass das volumenbezogene Verhältnis von umzusetzendem Citronellal zum Lösungsmittel etwa 1:1 bis etwa 1:20, bevorzugt von etwa 1:1 bis etwa 1:10 beträgt.

Es wurde gefunden, dass üblicherweise während der Reaktion ein Teil des Katalysatorkomplexes deaktiviert wird. Dies ist unter anderem auf Ligandenaustausch-Prozesse zwischen den jeweils eingesetzten Liganden der Formel (I), der eingesetzten Phenoxy-Aluminium-Verbindungen und dem durch Cyclisierung entstehenden Isopulegol zurückzuführen. Die deaktivierte Form des Katalysators ist, in Abhängigkeit von der Wahl der eingesetzten Lösemittel, üblicherweise in der Reaktionsmischung löslich.

Alternativ kann die eingesetzte Katalysatormenge so gewählt werden, dass der gesamte eingesetzte Katalysatorkomplex im Laufe bzw. nach Beendigung der erfindungsgemäßen Cyclisierungsreaktion deaktiviert ist. Dabei macht sich vorteilhaft bemerkbar, dass in diesem Falle aufgrund der vorstehend beschriebenen Ligandenaustausch-Prozesse der jeweils eingesetzte Ligand der Formel (I) ohne gesondert durchzuführende Hydrolyse freigesetzt wird.

Das erfindungsgemäße Verfahren eignet sich, wie bereits erwähnt, in gleichem Maße zur Cyclisierung von racemischem wie auch nicht-racemischem, d. h. optisch aktivem Citronellal zu racemischem wie nicht-racemischem Isopulegol.

Daher dient das erfindungsgemäße Verfahren in einer bevorzugten Ausführungsform zur Herstellung von optisch aktivem Isopulegol der Formel (IV.a) 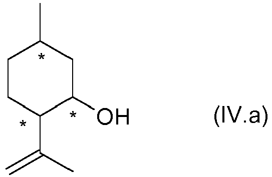 durch Cyclisierung von optisch aktivem Citronellal der Formel (V.a) 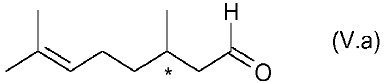 wobei (*) jeweils ein asymmetrisches Kohlenstoffatom bezeichnet.

Insbesondere dient das erfindungsgemäße Verfahren zur Herstellung von L-(-)-Ispulegol durch Cyclisierung von D-(+)-Citronellal.

Das so hergestellte racemische bzw. nicht-racemische Isopulegol stellt ein wertvolles Intermediat zur Herstellung von racemischem bzw. nicht-racemischem Menthol, einem der weltweit bedeutendsten Riech- bzw. Aromastoffe, dar. Menthol kann durch dem Fachmann an sich bekannte Methoden der Hydrierung, speziell der katalytischen Hydrierung an geeigneten Übergangsmetallkatalysatoren, wie beispielsweise in Pickard et al., J. Chem. Soc. 1920, 1253; Ohloff et al., Chem. Ber. 1962, 95, 1400; Pavia et al., Bull. Soc. Chim. Fr. 1981, 24, Otsuka et al., Synthesis 1991, 665 oder in der EP 1 053 974 A beschrieben, aus Isopulegol erhalten werden. Dabei bleibt bei geeigneter Wahl der Reaktionsbedingungen die relative bzw. absolute Konfiguration des eingesetzten Isopulegols weitgehend, in vielen Fällen vollständig erhalten.

Ein weiterer Gegenstand der vorliegenden Erfindung betrifft daher ein Verfahren zur Herstellung von Menthol umfassend die Schritte:
A) Herstellung von Isopulegol der Formel (IV) nacht dem erfindungsgemäßen Verfahren
B) Hydrierung der ethylenischen Doppelbindung des so erhaltenen Isopulegols.

In einer bevorzugten Ausführungsform dient dieses Verfahren zur Herstellung von optisch aktivem Menthol, speziell zur Herstellung von L-(-)-Menthol aus optisch aktivem L-(-)-Isopulegol.

Bezüglich der bevorzugten Ausführungsformen des erfindungsgemäßen Verfahrens zur Herstellung von Isopulegol wird im vollen Umfang auf die zuvor genannten Bevorzugungen verwiesen.

Die nachfolgenden Beispiele dienen dazu, die vorliegende Erfindung zu erläutern.

Gaschromatographische Analysen wurden nach der folgenden Methode durchgeführt: 50 m DB-WAX, ID.: 0,32 mm, FD.: 1,2 ym; 80 °C, 36- 230 °C, 15 °C/min auf 250 °C; t_{R} (Phenylcyclohexan): 16,5; t_{R} (Isopulegol): 13,7; t_{R} (Citronellal): 10,2.

Folgende HPLC-Methode wurde verwendet: CC250/4 Nucleodur C18 Gravity, 5 ym; C: Wasser - 0,05 % H₃PO₄; D: Acetonitril 20 : 80; Ausgang 93 bar, 25 °C; t_{R} (Phenylcyclohexan): 10,6; t_{R} (Isopulegol): 3,3; t_{R} (Diphenylphenol): 4,8. Konzentrationen der erhaltenen Reaktionsprodukte wurden GC- und HPLC-analytisch ermittelt.

### Beispiel 1: Verfahren zur Aufarbeitung von 2,6-Diphenylphenol

In einem ausgeheizten Kolben wurde 2,6-Diphenylphenol (25 g, 101,5 mmol) in wasserfreiem Toluol (731 ml) vorgelegt. Zu der klaren Lösung wurde bei Raumtemperatur eine Lösung von Triethylaluminium in Toluol (0,66 M, 18,2 ml, 33,8 mmol) gegeben. Die Lösung wurde für 1 h bei 25 °C gerührt. Die resultierende Katalysator-Lösung wurde auf 0 °C gekühlt und über einen Zeitraum von 3 h mit einem Gemisch aus Citronellal (258,7 g, 1,68 mol) und Brenztraubensäuremethylester (2,07 g, 0,02 mol) versetzt. Nach beendeter Zugabe wurde die Reaktionsmischung 3 h bei 0 °C und weitere 12 h bei Raumtemperatur nachgerührt. Das Lösungsmittel Toluol wurde über eine Kolonne (Durchmesser: 30 mm, Füllkörper 280 mm, Füllkörper 5 mm Metallringe, 100 mbar, Kopftemperatur 43 - 46 °C, Sumpftemperatur 48 - 85 °C) destillativ entfernt und das als Destillationsrückstand erhaltene Isopulegol wurde unter vermindertem Druck (6 mbar, Kopftemperatur 55 °C, Sumpftemperatur 75 °C) abdestilliert. Die erste Fraktion enthielt Isopulegol (38,6 g) in einer Reinheit von 98,4 %. Das im Destillationssumpf verbliebene Isopulegol wurde unter Zugabe von Phenylcyclohexan (52 g) unter vermindertem Druck (6 mbar, Kopftemperatur 53 - 54 °C, Sumpftemperatur 77 - 88 °C) abdestilliert. Die zweite Fraktion lieferte Isopulegol (224 g) in einer Reinheit von 90,1 %. Die Gesamtausbeute an isoliertem Isopulegol betrug 93 %. Das bei der Reaktion als Ligand eingesetzte 2,6-Diphenylphenol kristallisierte nach Abkühlen auf 25 °C innerhalb von 12 h aus dem öligen Sumpfprodukt aus. Die Suspension wurde über eine Glasfilternutsche abfiltriert und anschließend wurde der Filterkuchen bei einer Temperatur 95 °C und 3 mbar getrocknet. 2,6-Diphenylphenol wurde als kristalliner Feststoff (16,7 g) mit einer Ausbeute von 67 % erhalten. Dieser Feststoff, die Mutterlauge der Kristallisation sowie das Sumpfprodukt aus der destillativen Abtrennung wurden flüssigchromatographisch analysiert. Die Ergebnisse sind in Tabelle 1 zusammengestellt:

**Tabelle 1**

| | Isopulegol HPLC-Gew.% (FI.%) | 2,6-Diphenylphenol HPLC-Gew.% (FI.%) | Phenylcyclohexan HPLC-Gew.% (FI.%) |
|---|---|---|---|
| 2,6-Diphenylphenol | | 99,0 | |
| Destillationssumpf (62,9 g) | 2,1 9 (0,6) | 42,6 (73,43) | 40,92 (21,95) |
| Mutterlauge (32,5 g) | 2,73 (1,06) | 19,07 (48,0) | 56,02 (43,0) |

### Beispiel 2: Verfahren zur Aufarbeitung von 2,6-Diphenylphenol (umfassend die Hydrolyse des Sumpfprodukts)

In einem ausgeheizten Kolben wurde 2,6-Diphenylphenol (25 g, 101,5 mmol) in wasserfreiem Toluol (731 ml) vorgelegt. Zu der klaren Lösung wurde bei Raumtemperatur eine Lösung von Triethylaluminium in Toluol (0,66 M, 18,2 ml, 33,8 mmol) gegeben. Die Lösung wurde für 1 h bei 25 °C gerührt. Die resultierende Katalysator-Lösung wurde auf 0 °C gekühlt und über einen Zeitraum von 3 h mit einem Gemisch aus Citronellal (375 g, 2,43 mol) und Brenztraubensäuremethylester (3,75 g, 0,04 mol) versetzt.

Nach beendeter Zugabe wurde die Reaktionsmischung 3 h bei 0 °C und weitere 12 h bei Raumtemperatur nachgerührt. Das Lösungsmittel Toluol wurde über eine Kolonne (Durchmesser: 30 mm, Füllkörper 280 mm, Füllkörper 5 mm Metallringe, 100 mbar, Kopftemperatur 43 - 46 °C, Sumpftemperatur 48 - 85 °C) destillativ entfernt und das als Destillationsrückstand erhaltene Isopulegol wurde unter vermindertem Druck (6 mbar, Kopftemperatur 55 - 62 °C, Sumpftemperatur 75 - 115 °C) abdestilliert. Die erste Fraktion enthielt Isopulegol (341,9 g) in einer Reinheit von 98,4 %. Das im Destillationssumpf verbliebene Isopulegol wurde unter Zugabe von Phenylcyclohexan (40,5 g) unter vermindertem Druck (6 mbar, Kopftemperatur 72 °C, Sumpftemperatur 95 - 104 °C) abdestilliert. Die zweite Fraktion lieferte ein Gemisch enthaltend 56,9 % Isopulegol (26,7 g) und 39,9 % Phenylcyclohexan. Die Gesamtausbeute an isoliertem Isopulegol betrug 95 %.

Ein Teil des Sumpfprodukts der destillativen Auftrennung (26 g) wurde mit Phenylcyclohexan (653 g) verdünnt und mit einer 2%igen wässrigen NaOH-Lösung (104 ml) hydrolysiert. Die Suspension wurde 20 Minuten bei Raumtemperatur gerührt. Hierbei fiel das im Sumpfprodukt enthaltene Aluminium als Hydroxid aus. Nach der Phasentrennung wurde die organische Phase über Natriumsulfat getrocknet und filtriert. Das Phenylcyclohexan wurde destillativ (95 °C, 3 mbar) entfernt. 2,6-Diphenylphenol wurde als weißer Feststoff mit einer Ausbeute von 12,1 g erhalten.

Dieser Feststoff sowie das Sumpfprodukt aus der destillativen Abtrennung wurden flüssigchromatographisch analysiert. Die Ergebnisse sind in Tabelle 2 zusammengestellt:

**Tabelle 2**

| | Isopulegol HPLC-Gew.% (FI.%) | 2,6-Diphenylphenol HPLC-Gew.% (FI.%) | Phenylcyclohexan HPLC-Gew.% (FI.%) |
|---|---|---|---|
| 2,6-Diphenylphenol (12,1 g) | | 99,0 | |
| Destillationssumpf (26 g) | | 78,9 | 18,8 |

## Patentansprüche

1. Verfahren zur Aufarbeitung eines Aluminium-haltigen Reaktionsprodukts aus der Herstellung von Isopulegol durch Cyclisierung von Citronellal, enthaltend
i) Isopulegol,
ii) wenigstens einen Liganden der Formel (I), 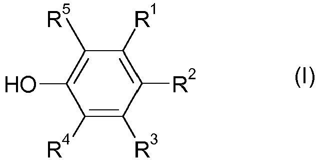 wobei
R¹, R², R³ jeweils unabhängig voneinander ausgewählt sind unter Wasserstoff, Halogen, Nitro, C₁-C₈-Alkyl, C₁-C₈-Alkoxy, Di(C₁-C₄-alkyl)amino und gegebenenfalls substituiertem oder unsubstituiertem Aryl, und
R⁴, R⁵ jeweils unabhängig voneinander ausgewählt sind unter Halogen, Nitro, C₁-C₈-Alkyl, C₁-C₈-Alkoxy, Di-(C₁-C₄-alkyl)amino und gegebenenfalls substituiertem oder unsubstituiertem Aryl oder Heteroaryl,
in freier und/oder komplexgebundener Form,
bei dem man
a) das Reaktionsprodukt ohne vorherige Hydrolyse einer destillativen Auftrennung unter Erhalt eines an Isopulegol angereicherten Kopfprodukts und eines an Isopulegol abgereicherten Sumpfprodukts unterzieht,
b) den Liganden der Formel (I) durch Kristallisation aus dem Sumpfprodukt abtrennt.

2. Verfahren gemäß Anspruch 1,
bei dem man
a) das Reaktionsprodukt ohne vorherige Hydrolyse einer destillativen Auftrennung unter Erhalt eines an Isopulegol angereicherten Kopfprodukts und eines an Isopulegol abgereicherten Sumpfprodukts unterzieht,
a.2) das an Isopulegol abgereicherte Sumpfprodukt mit einer wässrigen Base unter Erhalt einer Aluminium-haltigen wässrigen Phase und einer die Hauptmenge der Liganden der Formel (I) enthaltenden organischen Phase in innigen Kontakt bringt,
b) den Liganden der Formel (I) durch Kristallisation aus der organischen Phase abtrennt.

3. Verfahren gemäß einem der Ansprüche 1 oder 2, wobei das Reaktionsprodukt aus der Herstellung von Isopulegol durch Cyclisierung von Citronellal, zusätzlich ein niedriger siedendes Lösungsmittel (iii) enthält.

4. Verfahren gemäß einem der Ansprüche 1 bis 3, wobei das Reaktionsprodukt aus der Herstellung von Isopulegol durch Cyclisierung von Citronellal, zusätzlich einen Hilfsstoff (iv) enthält, der ausgewählt ist unter organischen Säuren, Carbonsäureanhydriden, Aldehyden, Ketonen und Vinylethern.

5. Verfahren gemäß einem der Ansprüche 1 bis 4, wobei vor der destillativen Auftrennung in Schritt a) aus dem Reaktionsprodukt zunächst gegebenenfalls enthaltenes Lösungsmittel und/oder Hilfsstoffe aus der Cyclisierung abgetrennt werden.

6. Verfahren gemäß einem der Ansprüche 1 bis 5, wobei man das Reaktionsprodukt vor und/oder während der destillativen Auftrennung in Schritt a) mit einem Lösungsmittel versetzt, dessen Siedepunkt unter den Bedingungen der Destillation um wenigstens 10 °C höher liegt als der Siedepunkt des Isopulegols.

7. Verfahren gemäß einem der Ansprüche 1 bis 6, wobei wenigstens einer der Verfahrensschritte kontinuierlich betrieben wird.

8. Verfahren gemäß einem der Ansprüche 2 bis 7, wobei man einen erwärmten Austrag des Sumpfprodukts aus Schritt a) mit einer erwärmten wässrigen Base in innigen Kontakt bringt und anschließend die Hauptmenge des Liganden aus der organischen Phase durch Kristallisation isoliert.

9. Verfahren gemäß einem der Ansprüche 1 bis 8, wobei das Aluminium-haltige Reaktionsprodukt erhalten wird bei der Herstellung von Isopulegol der Formel (IV) 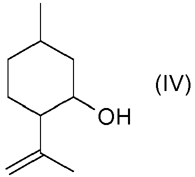 umfassend
die Cyclisierung von Citronellal der Formel (V) 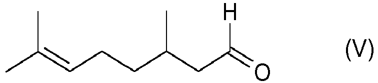 in Gegenwart eines tris(Phenyloxy)-Aluminium-Katalysator, der erhältlich ist durch Umsetzung eines Liganden der Formel (I), wie in Anspruch 1 definiert,
mit einer Aluminium-Verbindung der Formel (II),
(R¹⁴)₃₋ₚAlHₚ (II)
wobei
Al Aluminium bedeutet,
R¹⁴ einen verzweigten oder unverzweigten Alkylrest mit 1 bis 5 Kohlenstoffatomen und
p für 0 oder eine ganze Zahl von 1 bis 3 steht,
und/oder
mit einer Aluminium-Verbindung der Formel (III),
MAlH₄ (III)
wobei
Al Aluminium bedeutet und
M Lithium, Natrium oder Kalium bedeutet.

10. Verfahren gemäß Anspruch 9, wobei die Aluminium-Verbindung der Formel (II) ausgewählt ist unter Trimethyl- oder Triethylaluminium.

11. Verfahren gemäß einem der Ansprüche 9 oder 10 zur Herstellung von optisch aktivem Isopulegol der Formel (IV.a) 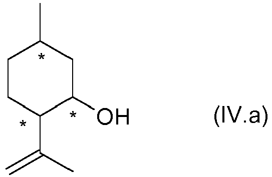 umfassend die Cyclisierung von optisch aktivem Citronellal der Formel (V.a) 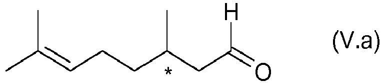 wobei (*) jeweils ein asymmetrisches Kohlenstoffatom bezeichnet.

12. Verfahren zur Herstellung von Menthol, umfassend die Schritte:
A) Herstellung von Isopulegol der Formel (IV) gemäß einem der Ansprüche 9 bis 11 und
B) Hydrierung der ethylenischen Doppelbindung des so erhaltenen Isopulegols.

## Claims

1. A method for working up an aluminum-containing reaction product from the production of isopulegol by cyclizing citronellal, comprising
i) isopulegol,
ii) at least one ligand of the formula (I), 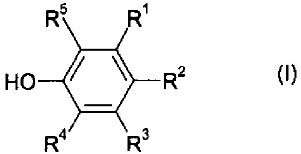 where
R¹, R², R³ are in each case independently of one another chosen from hydrogen, halogen, nitro, C₁-C₈-alkyl, C₁-C₈-alkoxy, di(C₁-C₄-alkyl)amino and optionally substituted or unsubstituted aryl, and
R⁴, R⁵ are in each case independently of one another chosen from halogen, nitro, C₁-C₈-alkyl, C₁-C₈-alkoxy, di(C₁-C₄-alkyl)amino and optionally substituted or unsubstituted aryl or heteroaryl,
in free and/or complex-bound form,
in which
a) the reaction product is subjected without prior hydrolysis to distillative separation to obtain an isopulegol-enriched top product and an isopulegol-depleted bottom product,
b) the ligands of the formula (I) are separated off from the bottom product by crystallization.

2. The method according to claim 1,
in which
a) the reaction product is subjected without prior hydrolysis to distillative separation to obtain an isopulegol-enriched top product and an isopulegol-depleted bottom product,
a.2)the isopulegol-depleted bottom product is brought into close contact with an aqueous base to give an aluminium-containing aqueous phase and an organic phase comprising the majority of the ligands of the formula (I),
b) the ligands of the formula (I) are separated off from the organic phase by crystallization.

3. The method according to one of claims 1 and 2, where the reaction product from the production of isopulegol by cyclizing citronellal additionally comprises a lower-boiling solvent (iii).

4. The method according to one of claims 1 to 3, where the reaction product from the production of isopulegol by cyclizing citronellal additionally comprises an auxiliary (iv) is chosen from organic acids, carboxylic anhydrides, aldehydes, ketones and vinyl ethers.

5. The method according to one of claims 1 to 4, where, prior to the distillative separation in step a), any solvent present and/or auxiliaries from the cyclization are firstly separated off from the reaction product.

6. The method according to one of claims 1 to 5, where, before and/or during the distillative separation in step a), the reaction product is admixed with a solvent whose boiling point, under the distillation conditions, is at least 10°C higher than the boiling point of isopulegol.

7. The method according to one of claims 1 to 6, where at least one of the process steps is operated continuously.

8. The method according to one of claims 2 to 7, where a heated discharge of the bottom product from step a) is brought into close contact with a heated aqueous base and then the majority of the ligand is isolated from the organic phase by crystallization.

9. The method according to one of claims 1 to 8, where the aluminum-containing reaction product is obtained in the production of isopulegol of the formula (IV) 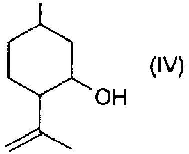 comprising
the cyclization of citronellal of the formula (V) 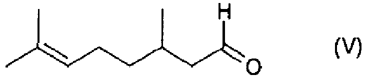 in the presence of a tris(phenyloxy)aluminum catalyst which is obtainable by reacting a ligand of the formula (I) as defined in claim 1,
with an aluminum compound of the formula (II),
(R¹⁴)₃₋ₚAlHₚ (II)
where
Alis aluminum,
R¹⁴ is a branched or unbranched alkyl radical having 1 to 5 carbon atoms and
p is 0 or an integer from 1 to 3,
and/or
with an aluminum compound of the formula (III),
MAlH₄ (III)
where
Alis aluminum and
M is lithium, sodium or potassium,

10. The method according to claim 9, where the aluminum compound of the formula (II) is chosen from trimethyl- or triethylaluminum.

11. The method according to one of claims 9 and 10 for producing optically active isopulegol of the formula (IV.a) 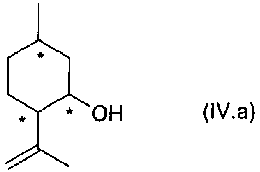 comprising the cyclization of optically active citronellal of the formula (V.a) 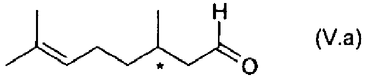 where (*) in each case refers to an asymmetric carbon atom.

12. A method of producing menthol comprising the steps:
A) production of isopulegol of the formula (IV) according to one of claims 9 to 11 and
B) hydrogenation of the ethylenic double bond of the isopulegol obtained in this way.

## Revendications

1. Procédé pour le retraitement d'un produit de réaction contenant de l'aluminium, provenant de la préparation de l'isopulégol par cyclisation de citronellal, contenant
i) de l'isopulégol,
ii) au moins un ligand de formule (I) 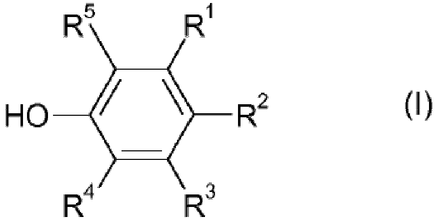 dans laquelle
R¹, R² et R³ sont chacun indépendamment des autres choisis parmi un atome d'hydrogène, un groupe halogéno, nitro, alkyle en C₁-C₈, alcoxy en C₁-C₈, di (alkyle en C₁₋C₄)amino, et aryle éventuellement substitué ou non-substitué, et
R⁴, R⁵ sont chacun indépendamment de l'autre choisis parmi un groupe halogéno, nitro, alkyle en C₁₋C₈, alcoxy en C₁-C₈, di (alkyle en C₁-C₄)amino, et aryle ou hétéroaryle éventuellement substitué ou non-substitué,
sous forme libre et/ou complexée,
dans lequel
a) on soumet le produit de réaction, sans hydrolyse préalable, à une séparation par distillation avec obtention d'un produit de tête enrichi en isopulégol et d'un produit de fond appauvri en isopulégol,
b) on sépare le ligand de formule (I) par cristallisation du produit de fond.

2. Procédé selon la revendication 1,
dans lequel
a) on soumet le produit de réaction, sans hydrolyse préalable, à une séparation par distillation avec obtention d'un produit de tête enrichi en isopulégol et d'un produit de fond appauvri en isopulégol,
a.2) on met en contact intime le produit de fond appauvri en isopulégol avec une base aqueuse, avec obtention d'une phase aqueuse contenant de l'aluminium et d'une phase organique contenant la quantité principale des ligands de formule (I),
b) on sépare le ligand de formule (I) par cristallisation de la phase organique.

3. Procédé selon l'une des revendications 1 ou 2, dans lequel le produit de réaction provenant de la préparation de l'isopulégol par cyclisation de citronellal contient en outre un solvant (iii) à bas point d'ébullition.

4. Procédé selon l'une des revendications 1 à 3, dans lequel le produit de réaction provenant de la préparation d'isopulégol par cyclisation de citronellal contient en outre une substance auxiliaire (iv), qui est choisie parmi les acides organiques, les anhydrides carboxyliques, les aldéhydes, les cétones et les vinyléthers.

5. Procédé selon l'une des revendications 1 à 4, dans lequel, avant la séparation par distillation dans l'étape a) à partir du produit de réaction, on sépare d'abord de la cyclisation les solvants et/ou substances auxiliaires éventuellement contenus.

6. Procédé selon l'une des revendications 1 à 5, dans lequel on ajoute au produit de réaction, avant et/ou pendant la séparation par distillation de l'étape a), un solvant dont le point d'ébullition est, dans les conditions de la distillation, d'au moins 10°C plus élevé que le point d'ébullition de l'isopulégol.

7. Procédé selon l'une des revendications 1 à 6, dans lequel au moins l'une des étapes du procédé est mise en oeuvre en continu.

8. Procédé selon l'une des revendications 2 à 7, dans lequel on met en contact intime avec une base aqueuse chauffée une émission chauffée du produit de fond de l'étape a), puis on isole de la phase organique par cristallisation la quantité principale du ligand.

9. Procédé selon l'une des revendications 1 à 8, dans lequel le produit de réaction contenant de l'aluminium est obtenu lors de la préparation de l'isopulégol de formule (IV) 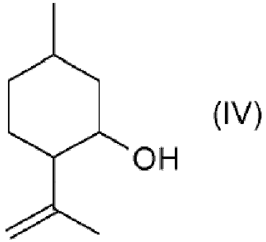 comprenant
la cyclisation de citronellal de formule (V) 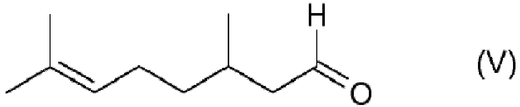 en présence d'un catalyseur à base de tris(phényloxy)-aluminium, qui peut être obtenu par réaction d'un ligand de formule (I) tel que défini dans la revendication 1
avec un composé de l'aluminium de formule (II)
(R¹⁴)₃₋ₚAlHₚ (II)
dans laquelle
Al désigne l'aluminium,
R¹⁴ représente un radical alkyle à chaîne droite ou ramifiée ayant 1 à 5 atomes de carbone, et
p vaut 0 ou est un nombre entier de 1 à 3,
et/ou
avec un composé de l'aluminium de formule (III)
MAlH₄ (III)
dans laquelle
Al désigne l'aluminium, et
M désigne le lithium, le sodium ou le potassium.

10. Procédé selon la revendication 9, dans lequel le composé de l'aluminium de formule (II) est choisi parmi le triméthyl- ou le triéthylaluminium.

11. Procédé selon l'une des revendications 9 ou 10 pour la préparation d'isopulégol optiquement actif de formule (IV.a) 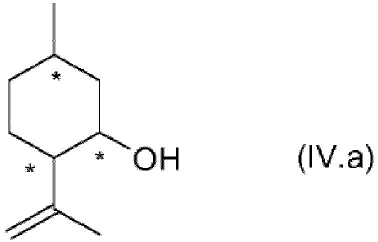 comprenant la cyclisation de citronellal optiquement actif de formule (V.a) 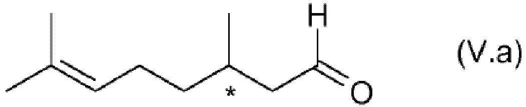 dans laquelle chaque (*) désigne un atome de carbone asymétrique.

12. Procédé de préparation de menthol, comprenant les étapes :
A) préparation d'isopulégol de formule (IV) selon l'une des revendications 9 à 11, et
B) hydrogénation de la double liaison éthylénique de l'isopulégol ainsi obtenu.
